Europäisches Patentamt

**European Patent Office**

Office européen des brevets

(19)

(11) Numéro de publication: **0 022 685**
**B1**

(12) **FASCICULE DE BREVET EUROPEEN**

(45) Date de publication du fascicule du brevet:
**25.01.84**

(51) Int. Cl.³: **C 12 N 15/00**

(21) Numéro de dépôt: **80400828.2**

(22) Date de dépôt: **09.06.80**

(54) Vecteurs pour le transfert et l'expression de matériel génétique et leurs applications.

(30) Priorité: **08.06.79 FR 7914806**

(43) Date de publication de la demande:
**21.01.81 Bulletin 81/3**

(45) Mention de la délivrance du brevet:
**25.01.84 Bulletin 84/4**

(84) Etats contractants désignés:
**BE CH DE GB IT LI**

(73) Titulaire: **INSTITUT PASTEUR, 25-28, rue du Docteur Roux, F-75724 Paris Cedex 15 (FR)**

(72) Inventeur: **Garapin, Florence, 23, rue Castagnary, F-75015 Paris (FR)**
Inventeur: **Garapin, Axel-Claude, 23, rue Castagnary, F-75015 Paris (FR)**

(74) Mandataire: **Gutmann, Ernest et al, Cabinet Plasseraud 84, rue d'Amsterdam, F-75009 Paris (FR)**

(56) Documents cités:
SCIENCE, vol. 203, 9 février 1979 L.W. ENQUIST et al.: "Cloning of herpes simplex type 1 DNA fragments in a bacteriophage lambda vector" pages 541-544
CHEMICAL ABSTRACTS, vol. 92, no. 15 14 avril 1980, réf.: 124694y, page 334, Columbus, Ohio, US C. WEISSMANN et al.: "Expression of cloned viral and chromosomal plasmid-linked DNA in cognate host cells"
NATURE, vol. 275, 19 octobre 1978 A.C.Y. CHANG et al.: "Phenotypic expression in E. coli of a DNA sequence coding for mouse di-hydrofolate reductase", pages 617-624
CHEMICAL ABSTRACTS, vol. 91, no. 23 3 décembre 1979, page 322, réf.: 189579p Columbus, Ohio, US S. SILVERSTEIN et al.: "Indentification of sequences coding for thymidine kinase in herpes simplex virus and transformed cell DNA"

(56) Documents cités: (suite)
Proc. Natl. Acad. Sci. USA, vol. 76, No. 8, août 1979 F. COLBERE-GARAPIN et al.: "Cloning of the active thymidine kinase gene of herpes simplex virus type 1 in Escherichia coli K-12" pages 3755-3759
GENE, 7, 1979 Elsevier, North-Holland Biomedical Press Amsterdam NL L.W. ENQUIST et al.: "Construction and characterization of a recombinant plasmid encoding the gene for the thymidine kinase of herpes simpelx type 1 virus", pages 335-342
NATURE, vol. 277, 11 janvier 1979 Stanford, California US R.C. MULLIGAN et al.: "Synthesis of rabbit beta-globin in cultured monkey kidney cells following infection with a SV40 beta-globin recombinant genome", pages 108-114
CHEMICAL ABSTRACTS, vol. 91, no. 3, 16 juillet 1979, page 396, réf.: 17471f Columbus, Ohio, US M. WIGLER et

(56) Documents cités: (suite)
al.: "Transformation of mammalian cells with genes
from procaryotes and eucaryotes"
CHEMICAL ABSTRACTS, vol. 75, no. 5, 2 août 1971, page
240, réf.: 32720z Columbus, Ohio, US W. MUNYON et al.:
"Transfer of thymidine kinase to thymidine kinaseless L
cells by infection with ultraviolet-irradiated herpes
simplex virus"
CHEMICAL ABSTRACTS, vol. 89, no. 17, 23 octobre
1978, page 222 réf.: 142263z Columbus, Ohio, US M.
WIGLER et al.: "Biochemical transfer of single-copy
eukaryotic genes using total cellular DNA as donor"

## Vecteurs pour le transfert et l'expression de matériel génétique et leurs applications

L'invention est relative à de nouveaux vecteurs dans le génome desquels peut être inséré, par fusion génétique, un fragment d'ADN étranger, susceptible de coder la production d'une protéine déterminée, procaryote ou eucaryote, ces vecteurs étant construits de façon telle qu'ils soient capables de transférer ce fragment d'ADN étranger dans une cellule eucaryote et d'induire l'expression dans celle-ci de ce fragment d'ADN étranger.

On a déjà décrit des vecteurs dans le génome desquels peut être inséré un fragment d'ADN étranger et qui sont aptes à transformer des cellules procaryotes, notamment des bactéries, à partir desquelles peut alors être isolée une protéine hybride contenant la protéine normalement codée par ce fragment d'ADN étranger dans son hôte naturel.

Parmi les applications envisagées de ces techniques, on peut citer la production de protéines ayant des vertus vaccinantes. La mise à contribution des cellules procaryotes, notamment de bactéries, en tant qu'«usine de production» de protéines de ce type, en quantité suffisante, suscite à ce jour bien des réserves de la part des spécialistes et des autorités médicales.

L'importance de ces réserves pourrait cependant être bien atténuée, si les cellules procaryotes du genre en question pouvaient être remplacées utilement par des cellules eucaryotes, notamment d'origine animale ou de préférence encore d'origine humaine, s'agissant de protéines vaccinantes qui seraient destinées à l'homme.

Cette hypothèse n'est pas à ce jour sans soulever a priori de très grandes difficultés. En effet, même si l'on fait abstraction des rendements de réplication, vraisemblablement plus réduits, des vecteurs qui pourraient être construits et modifiés par le fragment d'ADN approprié, une difficulté importante qui reste à surmonter est la détection et par conséquent l'isolement de celles des cellules eucaryotes dans lesquelles aurait effectivement été transféré un tel vecteur modifié parmi les cellules non affectées par ce même vecteur.

Certes, R.C. Mulligan et Coll. (Nature, Vol. 277, pages 108–114, 11 janvier 1979) ont montré qu'un génome recombinant résultant de l'insertion d'un ADN codant la β-globine de lapin dans l'ADN d'un virus SV 40, en lieu et place dans ce dernier du gène codant la majeure partie de la protéine de capside VP 1, pouvait être exprimé dans des cellules de rein de singe.

Ce génome recombinant avait été obtenu, par recombinaison génétique in vitro, d'une part, d'un fragment de 4,2 kilobases (kb), antérieurement obtenu à partir du virus SV 40, par digestions successives en présence des enzymes Hind III, EcoRI et BamH I et, d'autre part, d'un fragment codant la β-globine, préalablement extrait d'un plasmide qui avait été antérieurement décrit (pBR322-β-G2), après clivage séquentiel de ce dernier par Bgl II et Hind III.

Mais la réplication de ce recombinant et son expression dans des cellules eucaryotes (cellules de rein de singe) ont effectivement été réussies grâce au système de réplication exogène apporté, lequel avait conservé toute l'information apte à coder la réplication du virus SV 40, dont était bien connue auparavant la capacité d'infecter des cellules eucaryotes. Il va sans dire que le plasmide pBR322-β-G2, qui était lui-même dépourvu de l'origine de réplication adéquate, dans le système eucaryote considéré, n'aurait pas pu être utilisé comme vecteur pour transmettre efficacement, et surtout permettre, l'expression de la β-globine dans ces cellules eucaryotes.

En outre, les cellules infectées pouvaient aisément être repérées dans une culture en raison même du caractère viral de ce génome recombinant, du fait des modifications induites par celui-ci dans les conditions de croissance des cellules infectées.

Parmi les inconvénients d'un tel système, il faut naturellement souligner l'oncogénicité de ce recombinant, tout aussi incompatible a priori avec le type d'application envisagé que les inconvénients mentionnés plus haut à propos des bactéries en tant qu'hôte producteur de protéines.

L'invention a pour but de remédier au moins en partie à de telles difficultés, notamment de fournir des vecteurs susceptibles de transférer à l'intérieur de cellules-hôtes eucaryotes un fragment d'ADN alors susceptible d'être exprimé dans ces cellules sans transformation aussi importante, sinon en l'absence de modifications sensibles de leurs conditions de croissance.

Elle a également pour but un procédé de mise en œuvre d'un tel vecteur permettant, lorsqu'il est utilisé pour transférer un fragment d'ADN déterminé dans certains types de cellules eucaryotes, la détection aisée de celles des cellules dans lesquelles ce fragment d'ADN se trouvera effectivement exprimé.

Le vecteur selon l'invention, dérivé du plasmide pBR322, est caractérisé par la combinaison des deux facteurs de résistance à l'ampicilline et à la tétracycline respectivement, normalement contenus dans pBR322, d'une part, et d'un ADN de complémentation contenant un gène de thymidine-kinase, tel que celui du virus de l'herpès, inséré dans le site Pvu II du plasmide, d'autre part. Au gène de la thymidine-kinase contenu dans le vecteur selon l'invention, correspond normalement, dans au moins certaines catégories de cellules eucaryotes, un gène homologue exprimable sous la forme d'une protéine homologue à la thymidine-kinase. La déficience, naturelle ou induite, desdites cellules eucaryotes en ce gène homologue est alors susceptible d'être complémentée par l'ADN «étranger» contenu dans le vecteur selon l'invention, après l'introduction de celui-ci dans de telles cellules (d'où l'utilisation de l'expression «ADN de complémentation» pour le désigner).

L'adjectif «étranger» dont il est fait usage dans l'expression «ADN étranger» vise à faire ressortir que le vecteur selon l'invention est un recombinant dont l'ADN étranger ne forme qu'un des éléments constitutifs du vecteur, les autres éléments constitutifs de ce vecteur étant dépourvus d'une origine de réplication exploitable par la machinerie cellulaire des cellules, notamment eucaryotes, dans lesquelles il est destiné à être transféré. Ils peuvent naturellement être pourvus d'une origine de réplication non eucaryote ne modifiant pas les autres fonctions habituelles cellulaires.

Il est encore entendu que, dans le cadre du présent exposé, on entend par le terme «complémentation» la compensation de la déficience susdite, telle qu'elle se manifeste quand lesdites cellules antérieurement déficientes deviennent capables, lorsque placées dans un milieu de culture dans lequel cette déficience leur aurait normalement interdit de pousser, de se développer à nouveau après incorporation préalable du fragment d'ADN du genre en question à leur patrimoine génétique.

Le fragment d'ADN contenant le gène de la thymidine-kinase du virus herpès simplex HSV-1 constitue un ADN de complémentation particulièrement efficace. Ce fragment peut être excisé du génome du virus par des enzymes de restriction spécifiques, tels que Bam HI. A ce gène de la thymidine-kinase d'origine virale correspondent, dans de nombreux types de cellules eucaryotes, des gènes homologues propres à diriger la synthèse de la thymidine-kinase (enzyme phosphorylant la thymidine).

La possibilité de suppléer, par l'introduction préalable du gène entier de la thymidine-kinase du virus de l'herpès dans des cellules de souris du type L antérieurement déficientes, en raison d'une mutation induite ou provoquée affectant leur gène de la thymidine-kinase endogène, a déjà été décrite par M. Wigler et Coll. (Cell. Vol. II, 223–232, 1977). Les cellules déficientes, dites TK$^-$, sont sélectionnées en raison de leur incapacité de synthétiser la thymidine-kinase et de pousser dans un milieu tel que celui connu sous le nom de «milieu Hat» (contenant de l'hypoxanthine et de l'aminoptérine en sus de la thymidine). Ce milieu est connu pour n'autoriser l'éventuelle synthèse de thymidine phosphate que par l'intermédiaire d'une voie métabolique dite «voie de sauvetage» («salvation pathway»), cette voie impliquant cependant que soit préservée l'intégrité du gène TK des cellules susceptibles de s'y développer. Des cellules TK$^-$ deviennent néanmoins capables de se développer dans ce même milieu dès lors qu'elles ont été modifiées par incorporation du gène TK du virus de l'herpès dans leur patrimoine génétique transmissible par ces cellules à leur descendance, comme suite à leurs divisions cellulaires successives. De «TK$^-$» qu'elles étaient antérieurement, avant la susdite incorporation, ces cellules sont alors devenues «TK$^+$», du fait de la restauration alors constatée de leur capacité de phosphoryler la thymidine dans le susdit milieu et, par conséquent, de s'y développer.

La réplication du vecteur selon l'invention – et l'expression du gène de la thymidine-kinase, inséré dans ce vecteur – dans des cellules eucaryotes n'impliquent donc plus la présence antérieurement nécessaire d'un système de réplication exogène (tel que celui du virus SV 40 susmentionné). Elles sont alors, en raison du caractère homologue de l'ADN TK du vecteur, sous la dépendance directe du système de réplication propre à ces cellules eucaryotes, d'où l'absence de caractère «transformé» de ces cellules qui ont reçu un gène d'origine virale.

La détection au sein d'une population cellulaire de celles des souches auxquelles le vecteur selon l'invention se trouve être incorporé est également aisée, notamment par l'intermédiaire de techniques immunologiques mettant en œuvre des anticorps antivirus de l'herpès, ceux-ci étant alors aptes à déceler les souches rendues TK$^+$ par le vecteur selon l'invention, alors que les souches TK$^+$ naturelles ne sont pas affectées.

L'ADN de complémentation peut naturellement comporter un gène naturel. Il peut aussi être synthétisé, notamment de façon enzymatique, par copie d'un ARN messager correspondant.

Les parties constitutives du vecteur selon l'invention, autres que l'ADN de complémentation, qui n'apparaîtront pas phénotypiquement dans des tests de reconnaissance immunologiques tels que mentionnés ci-dessus, proviennent de pBR322. Il est pourvu de sites déterminés permettant l'insertion dans le vecteur – et son expression future – d'un «ADN producteur» déterminé correspondant à la protéine dont la production est recherchée. Ces sites sont notamment clivables par des endonucléases déterminées. Plusieurs sites de ce type n'ont pas leur équivalent dans l'ADN de complémentation lui-même, de sorte que l'insertion en phase (le cas échéant par l'intermédiaire d'une chaîne de nucléotides comprenant un ou deux nucléotides, le cas échéant augmenté d'un nombre entier de triplets) d'un ADN producteur partiellement incomplet ou d'un ADN producteur complet dans ce site n'affectera pas l'ADN de complémentation. Celui-ci restera alors apte, en tout état de cause, à pourvoir, d'une part, à sa fonction de direction de la réplication sous le contrôle de la cellule eucaryote, d'autre part, à sa fonction de «marqueur» dans ces cellules.

Ces parties du vecteur de l'invention comprennent les éléments d'ADN qui leur sont nécessaires pour également permettre le clonage de ce vecteur dans des micro-organismes, notamment des bactéries telles que E. coli. Grâce à l'insertion du gène de la thymidine-kinase dans le site pVU II de pBR322, le vecteur selon l'invention comprend deux marqueurs, plus particulièrement des facteurs de résistance à l'ampicilline et à la tétracycline, aptes à faciliter, de façon en soi connue, le repérage de celles des colonies de micro-organismes effectivement transformées. Chacun de ces marqueurs, à l'extérieur de l'ADN de complémentation, permet la détection de ceux des micro-organismes, notamment de celles des bactéries qui sont aptes à héberger – et propager –

ce même vecteur, ne serait-ce que pour en produire des quantités suffisamment importantes, en vue de son utilisation ultérieure dans des cultures de cellules eucaryotes. Il possède en outre plusieurs sites de coupure uniques (BamH I, Hind III, Sal I) dans le second facteur, de sorte que l'intégration d'un ADN dans l'un de ces sites causera la perte partielle ou totale de la résistance à la tétracycline. Ce vecteur est particulièrement efficace pour réaliser le clonage de matériel génétique supplémentaire, notamment dans E. coli, préalablement à son utilisation pour la transformation de cellules eucaryotes.

Le procédé selon l'invention, pour la détection aisée des vecteurs comportant un ADN de complémentation tel qu'il a été défini plus haut, est caractérisé en ce qu'on l'incorpore à une culture de cellules eucaryotes déficientes en le gène homologue de cet ADN de complémentation (par exemple suite à une mutation accidentelle ou provoquée), ces cellules appartenant à une espèce normalement pourvue d'un tel gène homologue, celui-ci étant antigéniquement distinct de celui de l'ADN de complémentation, et en ce que l'on produit une culture de ces cellules dans un milieu dans lequel celles qui n'ont pas incorporé le vecteur selon l'invention ne peuvent pas se développer.

Il résulte déjà de ce qui précède que les souches ayant intégré le vecteur selon l'invention présentent toutes les caractéristiques qui permettront leur identification aisée dans une population cellulaire, d'où les possibilités de clonage des cellules ayant incorporé à leur patrimoine génétique le vecteur en question. Il en va évidemment de même si ce vecteur a au préalable été modifié, par insertion dans le site approprié d'un ADN producteur déterminé, d'où encore d'ailleurs des possibilités d'identification supplémentaires des souches modifiées (par exemple par hybridation in situ ou directement expression de la protéine recherchée correspondante). Le vecteur décrit ci-dessus et l'ADN producteur peuvent être transférés dans la cellule eucaryote sous forme linéaire ou circulaire, les deux ADN étant ligués ou non.

D'autres caractéristiques de l'invention apparaîtront encore au cours de la description qui suit de la construction de vecteurs préférés et des conditions dans lesquelles ils peuvent être utilisés pour transférer dans une cellule eucaryote un ADN producteur spécifiant une protéine déterminée. Référence sera faite aux dessins dans lesquels:

— la fig. 1 est un schéma simplifié du plasmide pBR322,

— la fig. 2 est une carte de restriction d'un fragment d'ADN issu du génome du virus de l'herpès, souche F HSV-1, et contenant le gène de la thymidine-kinase.

1. Préparation du virus de l'herpès, souche F HSV-1.

Des cellules humaines diploïdes MRC5 sont cultivées dans un milieu de Eagle contenant du sérum de veau (10%) et 3,6 g/l de tricine. La souche de virus susdite est propagée dans cette culture de cellules à raison d'un taux d'infection de 0,1 unité formatrice de plages par cellule. La culture est récoltée 48 heures après infection. La suspension de virus extracellulaire obtenue est clarifiée à 2.000 g pendant 10 minutes et conservée à −70°C.

2. Purification de l'ADN viral.

La suspension clarifiée de virus est soumise à centrifugation à 60.000 g pendant une heure à 4°C. Le culot récupéré est déprotéinisé avec de la protéinase K (MERCK) à raison de 100 µg par ml à 37°C, pendant deux heures. Cette opération est ensuite répétée, cette fois-ci en présence de dodécyl-sulfate de sodium (0,3%). L'ADN viral est séparé de l'ADN cellulaire en présence de bromure d'«éthidium» par centrifugation différentielle dans un gradient de densité dans une solution d'iodure de sodium. Le bromure d'éthidium est séparé de la fraction contenant l'ADN viral par des extractions avec de l'alcool isoamylique. Après des dialyses répétées contre un tampon Tris.HCL 20 mM; pH 7,5; EDTA 1 mM, on obtient un ADN viral caractérisé par un rapport $A_{260}:A_{280}$ supérieur à 1,90.

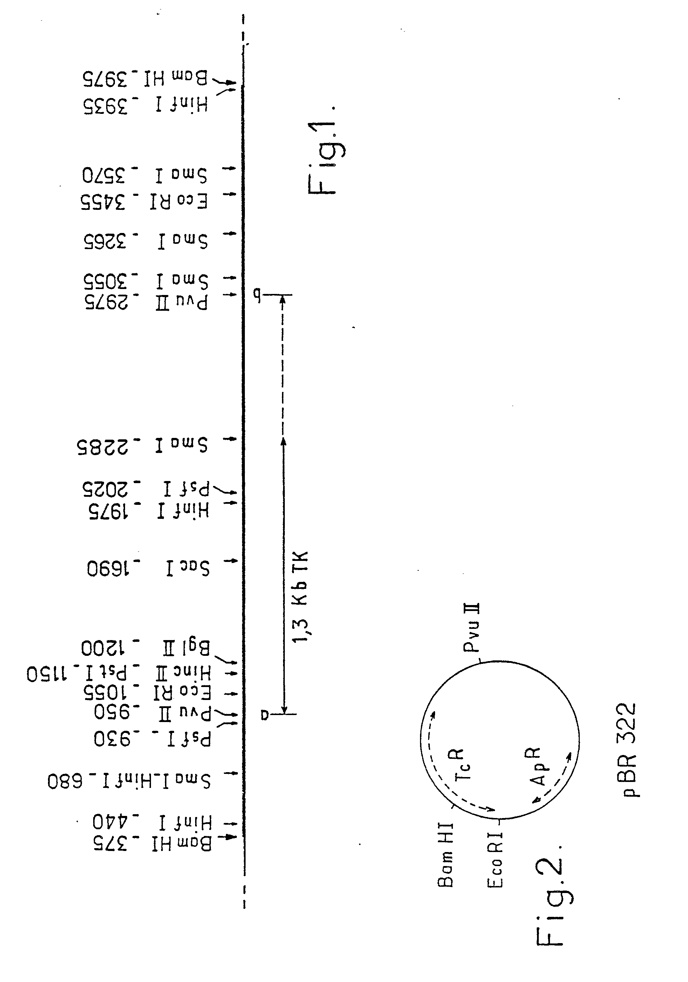

La fig. 1 est une carte de restriction de la partie de l'ADN viral contenant le gène TK dont l'emplacement relatif est montré par le segment en trait plein «1,3 Kb TK».

Par recombinaison génétique, et en mettant en œuvre l'enzyme de restriction Pvu II, on peut réaliser l'insertion du fragment d'ADN plus petit repéré par les lettres a et b dans la fig. 1, mais comportant encore la totalité du gène TK dans le site Pvu II du plasmide pBR322, représenté à la fig. 2.

3. Construction du recombinant pAGO.

L'ADN viral, d'une part, et le plasmide pBR322 (dont on a schématiquement représenté dans la fig. 2 les positions relatives des sites de résistance à l'ampicilline ($Ap^R$) et à la tétracycline ($Tc^R$), ainsi que du site Pvu II), d'autre part, sont respectivement traités comme suit avec l'enzyme de restriction Pvu II selon les conditions propres à cette enzyme. Les réactions sont terminées par chauffage pendant 5 minutes à 65°C.

Suite à la ligation des fragments d'ADN en présence de T4 DNA ligase au sein d'une solution tampon contenant, par 100 microlitres: Tris.HCl 20 mM; pH 7,4; 2,6 mg d'albumine bovine; dithiothreitol 5,2 mM; ATP 0,05 mM et 2µl d'enzyme par µg d'ADN. Ces opérations de ligation sont effectuées pendant la nuit à 4°C.

4. Sélection et amplification de vecteurs conformes à l'invention.

On procède comme décrit ci-après à la transformation d'une culture de cellules E. coli 1106 $r^-_K m^-_K$.

Une culture préparée pendant la nuit précédente des bactéries ci-dessus est diluée dans 100 ml d'un bouillon de culture L et incubée à 37°C jusqu'à ce que soit atteinte une absorbance à 600 nm de 0,4. Les cellules sont ensuite centrifugées, récupérées et remises en suspension dans 50 ml d'une solution de CaCl₂ 50mM. Les cellules sont à nouveau centrifugées, récupérées et remises en suspension dans 4 ml de CaCl₂ 50 mM et conservées dans la glace.

50 microlitres d'ADN récupérés dans l'opération précédente sont mélangés avec 150 microlitres de la suspension bactérienne. Les cellules sont ensuite maintenues à 45 °C pendant 3 minutes, puis à 37 °C pendant 5 minutes. Selon une variante, les cellules peuvent aussi être maintenues 30 minutes sur la glace, puis mises à 37 °C pendant 5 minutes. On ajoute 0,5 ml de bouillon L et l'on agite les cellules à 37 °C pendant 45 minutes.

On procède ensuite à l'isolement tant des bactéries hébergeant un plasmide du type pBR322 que des bactéries hébergeant des plasmides du type pAGO.

Les plasmides répliqués dans les dernières souches correspondantes, sont récupérés à partir de ces bactéries par des techniques tout à fait classiques comprenant la rupture des bactéries, la récupération des produits intracellulaires et la séparation des autres constituants du type ADN par centrifugation différentielle dans un gradient de densité en présence de bromure d'«éthidium».

Le recombinant pAGO contient le gène TK⁺ (ou un ADN équivalent susceptible de l'exprimer), plus particulièrement le fragment délimité par deux extrémités Pvu II, de part et d'autre du gène TK (voir fig. 1), dont la taille est de l'ordre de 1,3 kb.

5. Transfert du plasmide dans des cellules de souris L TK⁻.

On opère selon la technique décrite par Graham et Van der Ed (1973) avec quelques modifications. Le plasmide est mélangé avec un ADN de sperme de saumon utilisé comme entraîneur à une concentration finale de 10 microgrammes par ml dans un tampon isotonique Hepes: NaCl 8 g/l; KCl 0,37 g/l; Na₂HPO₄ 0,125 g/l; dextrose 1 g/l et Hepes 5 g/l (pH 7,05).

On ajoute encore le produit dénommé Aprotinine (SIGMA) à raison de 0,2 ml/ml de solution d'ADN. On ajoute aussi CaCl₂ jusqu'à une concentration finale de 125 mM. On laisse reposer à température ambiante de 5 à 10 minutes.

Des cellules de souris L TK⁻ cultivées dans du milieu de Leibovitz L 15 contenant 10% de sérum de veau et 3,6 g/l de tricine, sont prélevées et mises dans des boîtes de Corning de 25 cm². Les cultures de 24 heures sont recouvertes d'un mélange de 0,5 ml d'ADN et de phosphate de calcium. Après 30 minutes à 37 °C on ajoute 10 ml du milieu de culture. 6 à 7 heures plus tard les cultures cellulaires sont agitées avec 20% de DMSO selon la méthode de Stow et Coll. (1976). 24 heures plus tard le milieu est changé. Il est remplacé par le milieu d'Hat de Munyon déjà cité.

On alimente les boîtes avec du milieu Hat tous les 4 à 7 jours. Les premières colonies de cellules transformées apparaissent 8 à 10 jours plus tard.

Trois semaines après le début de l'expérience, on récupère des colonies de cellules devenues TK⁺.

La présence de protéines exprimées par le gène TK est également détectée par la technique déjà décrite par Munyon.

6. Utilisation du plasmide selon l'invention.

Le vecteur selon l'invention, dont un exemple vient d'être décrit, peut être utilisé pour l'insertion et/ou le transfert d'un ADN choisi. Il est avantageusement introduit dans un site de restriction par exemple Hind III, Sal I, au besoin après modification de ses extrémités cohésives, dans les conditions déjà rappelées plus haut. D'autres sites du plasmide, en dehors du gène TK, peuvent également, le cas échéant, être mis en œuvre dans les conditions indiquées plus haut.

Le vecteur selon l'invention et l'ADN producteur peuvent être transférés dans la cellule eucaryote sous forme linéaire ou circulaire, les deux ADN étant ligués ou non.

Le vecteur modifié peut être sélectionné et utilisé pour le transfert de l'ADN producteur dans une souche de cellules eucaryotes en mettant également en jeu les techniques qui ont été décrites dans ce qui précède.

A titre d'exemple d'ADN producteurs qui peuvent être insérés dans le vecteur selon l'invention, on citera: les gènes de la somatostatine, lysozyme de poule, ou un ADN codant l'antigène de l'hépatite virale.

L'invention concerne également les souches de cellules eucaryotes ou procaryotes hébergeant les vecteurs tels qu'ils ont été définis, y compris ceux comportant, inclus dans leur génome, un ADN producteur.

Dans le cas des vecteurs pour lesquels le gène TK est lié directement à un promoteur procaryote (par exemple celui du gène β-galactosidase), un haut niveau d'expression du gène TK viral peut être obtenu dans la bactérie. En plus des propriétés vaccinantes éventuelles, cette protéine virale peut être utilisée pour le diagnostic, notamment par recherche d'anticorps antiviraux dans le sérum du malade.

L'invention concerne encore tout vecteur conforme aux définitions sus-indiquées qui, ligué ou non à un ADN producteur et cotransféré avec celui-ci dans la cellule eucaryote, permet de reconnaître et/ou de sélectionner les cellules ayant reçu le vecteur et par conséquent l'ADN producteur.

**Revendications**

1. Vecteur dérivé du plasmide pBR322, caractérisé par la combinaison des deux facteurs de résistance à l'ampicilline et à la tétracycline respectivement, normalement contenus dans pBR322, d'une part, et d'un ADN de complémentation contenant un gène de thymidine kinase, tel que celui du virus de l'herpès, inséré dans le site Pvu II du plasmide, d'autre part.

2. Vecteur selon la revendication 1, caractérisé en ce que l'ADN de complémentation est constitué par un fragment du gène de la thymidine kinase du virus de l'herpès, délimité par des extrémités Pvu II et ayant une longueur de l'ordre de 1,3 kilobases.

3. Vecteur selon la revendication 1 ou la revendication 2, caractérisé par la présence dans son génome, extérieurement à l'ADN de complémentation, d'un ADN producteur d'insertion, codant pour une protéine dont la production est recherchée.

4. Application du vecteur selon la revendication 3 à la transformation et à la détection de cellules eucaryotes aptes à exprimer la protéine pour laquelle code l'ADN producteur, comprenant la réalisation d'une culture de cellules eucaryotes déficientes en leur gène de thymidine kinase endogène, au sein d'un milieu dans lequel la susdite déficience leur interdirait normalement de pousser, et en ce que l'on produit le clonage des cellules qui se sont développées grâce à l'incorporation à leur patrimoine génétique du vecteur en question.

## Claims

1. Vector derived from plasmid pBR322, characterized by the combination of two resistance factors to ampicilline and to tetracycline respectively, normally contained in pBR 322, on the one hand, and of a complementation DNA containing a gene of thymidin kinase, such as that of the herpes virus, inserted within the Pvu II site of the plasmid, on the other hand.

2. Vector according to claim 1, characterized in that the complementation DNA is constituted by a fragment of the gene of thymidin kinase of the virus of herpes, bounded by Pvu II extremities and having a length of about 1.3 kilobases.

3. Vector according to claim 1 or to claim 2, characterized by the presence in its genome, outside from said complementation DNA, of an insert of producer DNA coding for a protein the production of which is sought.

4. Use of the vector according to claim 3 for the transformation and detection of eucaryotic cells capable of expressing the protein which is encoded by the producing DNA, which comprises the carrying out of a culture of eucaryotic cells having a deficiency as concerns their endogenous gene of thymidin kinase within a medium in which said deficiency would normally prevent them from growing and in that one produces the cloning of the cells which have grown owing to the incorporation into their genetic patrimony of the said vector.

## Patentansprüche

1. Von Plasmid pBR322 abgeleiteter Vektor, gekennzeichnet einerseits durch die Kombination der zwei Widerstandsfaktoren gegen Ampicillin und Tetracyclin, die normalerweise in pBR322 enthalten sind, und anderseits durch ein ADN-Komplement, das ein Gen der Thymidinkinase enthält, wie dasjenige des Herpesvirus, eingefügt in die Stelle Pvu II des Plasmids.

2. Vektor nach Anspruch 1, dadurch gekennzeichnet, dass das ADN-Komplement aus einem Fragment des Gens der Thymidinkinase des Herpesvirus aufgebaut ist, begrenzt durch die Enden Pvu II und mit einer Länge in der Grössenordnung von 1,3 Kilobasen.

3. Vektor nach Anspruch 1 oder 2, dadurch gekennzeichnet, dass er in seinem Genom, ausserhalb des ADN-Komplements, einen ADN-Erzeuger der Einfügung enthält, der für ein Protein kodiert ist, dessen Erzeugung gewünscht ist.

4. Verwendung des Vektors nach Anspruch 3 zur Transformation und zur Bestimmung der eukaryontischen Zellen, die fähig sind, das Protein für den Kode des ADN-Erzeugers auszudrücken, gekennzeichnet durch die Bereitstellung einer Kultur von eukaryontischen Zellen mit einem Mangel an ihrem endogenen Thymidinkinase-Gen in einem Nährmedium, welches normalerweise die Förderung des genannten Mangels verhindert, und dadurch, dass man einen Klon der Zellen erzeugt, die sich Dank des Einbaues in ihr genetisches Erbgut des in Frage stehenden Vektors entwickeln.

Fig.1.

BamHI – 375
HinfI – 440
SmaI,HinfI – 680
PsfI – 930
PvuII – 950
EcoRI – 1055
HincII
PstI – 1150
BglII – 1200
SacI – 1690
HinfI – 1975
PsfI – 2025
SmaI – 2285
PvuII – 2975
SmaI – 3055
SmaI – 3265
EcoRI – 3455
SmaI – 3570
HinfI – 3935
BamHI – 3975

a
b
1,3 Kb TK

Fig.2.

BamHI
EcoRI
TcR
ApR
PvuII

pBR 322